# EUROPEAN PATENT APPLICATION

(11) **EP 4 279 514 A1**
(43) Date of publication of application: **22.11.2023**
(21) Application number: 21918937.0
(22) Date of filing: 03.09.2021
(51) Int. Cl.: C07K 19/00, G01N 33/66

(54) **FUSION ENZYME AND USE THEREOF IN PAPER-BASED BIOSENSOR**

(30) Priority: 12.01.2021 CN 202110038511
(71) Applicant: Biology Institute Of Shandong Academy Of Sciences, Jinan, Shandong 250014 (CN)
(72) Inventor: GONG, Weili, Jinan, Shandong 250014 (CN); MA, Yaohong, Jinan, Shandong 250014 (CN); WANG, Binglian, Jinan, Shandong 250014 (CN); LIU, Qingai, Jinan, Shandong 250014 (CN); ZHENG, Lan, Jinan, Shandong 250014 (CN); LI, Qiushun, Jinan, Shandong 250014 (CN); HAN, Qingye, Jinan, Shandong 250014 (CN); CAI, Lei, Jinan, Shandong 250014 (CN); MENG, Qingjun, Jinan, Shandong 250014 (CN); YANG, Yan, Jinan, Shandong 250014 (CN)
(74) Representative: Huang, Liwei
(86) International application number: PCT/CN2021/116537
(87) International publication number: WO 2022/151746

(57) **Abstract**

Provided are a fusion enzyme and use thereof in a paper-based biosensor. A GDH-linker-CBM fusion enzyme contructed by a flavin adenine dinucleotide (FAD)-dependent glucose dehydrogenase (GDH) gene and a carbohydrate binding module (CBM) gene is prepared into a paper-based sensor by means of the modification effect of the carbohydrate binding module and a linker peptide.

## Description

### TECHNICAL FIELD

The present invention belongs to the technical field of biological enzyme genetic engineering and biosensing, and particularly relates to a GDH-linker-CBM fusion enzyme constructed by a flavin adenine dinucleotide (FAD)-dependent glucose dehydrogenase (GDH) gene and a carbohydrate binding module (CBM) gene, and use of the fusion enzyme in a paper-based biosensor.

### BACKGROUND

Disclosing the information in this Background section is only intended to increase understanding of the general background of the present invention, and is not necessarily to be taken as an acknowledgement or any form of suggestion that this information constitutes the prior art that is already well known to those of ordinary skill in the art.

Strict control of physiological blood glucose concentration is the only way for diabetics to avoid life-threatening complications, and therefore blood glucose monitoring is an important component of diabetes management. Currently in developed countries with well-established healthcare infrastructures, blood glucose control through blood glucose self-monitoring has become an economical and effective way for the public, but in many resource-poor countries, many fatal diseases cannot be dealt with due to lack of laboratory infrastructures and trained professionals, and diabetes care is usually not given priority. Therefore, the development of a simple, low-cost, easy-to-operate, and portable glucose sensor becomes an urgent need. In recent years, paper-based microfluidic systems have received considerable attention in the development of simple, low-cost, and portable glucose detection devices. Its advantages include: (1) oxidoreductase can be immobilized directly into a paper substrate by physical adsorption; (2) many microfluidic elements can be integrated on paper with complex 3D microfluidic channels; and (3) a wide variety of electrode materials can be easily screen-printed onto paper without the need for expensive clean room facilities.

A glucose sensor for blood glucose monitoring typically employs glucose oxidase as a biological recognition element, but glucose oxidase is susceptible to oxygen partial pressure, and thus the glucose sensor has a great limitation in detecting samples with significant differences in oxygen content such as venous blood, arterial blood and samples from high altitude areas. Glucose dehydrogenase (GDH) with flavin adenine dinucleotide (FAD) as a coenzyme does not use oxygen as an electron acceptor and has high enzymatic activity, high substrate specificity, and good stability, and becomes a research hotspot enzyme molecule for the development of a portable glucose sensor. However, at present, GDH is generally randomly immobilized on the surface of an electrode by means of physical adsorption or covalent binding, and an active center of an enzyme molecule is often hidden or not properly oriented, resulting in a large loss of enzyme activity, and poor detection sensitivity of the portable glucose sensor.

In recent years, immobilization of enzymes by biological affinity adsorption has attracted more and more attention, with the greatest advantages of specific binding of the enzyme molecule to a carrier material, a controlled immobilization direction and a minimal conformational change of the enzyme molecule. Carbohydrate-binding modules (CBMs) are natural carbohydrate active enzyme molecules that have no catalytic activity themselves, but can have specific binding domains to polysaccharides such as cellulose. With the development of techniques such as molecular biology, synthetic biology, and computer-aided simulation, researchers currently use a fusion DNA technology to attempt to obtain a wide variety of fusion proteins of CBM and the enzyme molecule to improve the affinity of the enzyme molecule to a substrate, the stability of the enzyme molecule, or the enzyme activity of the enzyme molecule. However, a transformation strategy of improving the detection sensitivity of an enzyme electrode sensor by using the specific affinity adsorption characteristics of CBM to cellulose to construct a fusion enzyme, and using a paper base as an immobilized carrier of the fusion enzyme molecule is hardly reported at home and abroad.

### SUMMARY

In view of the above research status, the inventors believe that providing a paper-based sensor based on glucose dehydrogenase helps to obtain a blood glucose monitoring device with excellent performance, which is a promising transformation direction. Based on this research idea, in the present invention, a fusion enzyme is constructed by using the specific affinity adsorption characteristics of CBM to cellulose, and a paper base is used as an immobilized carrier of the fusion enzyme molecule. After determination, the optimized fusion enzyme not only shows good affinity to a paper-based carrier, but also improves the detection sensitivity of an enzyme electrode sensor from several aspects.

Based on the above technical effects, the present invention provides the following technical solutions:
according to a first aspect of the present invention, provided is a fusion enzyme, including a peptide fragment of glucose dehydrogenase (GDH) and a peptide fragment of a carbohydrate binding module (CBM), the two parts being linked by a flexible peptide chain.

Based on the above design idea, the fusion enzyme is obtained in the present invention, the binding force of glucose dehydrogenase to a cellulose paper base can be effectively increased through the linking action of the carbohydrate binding module (CBM), and it is expected to obtain a paper-based sensor with higher detection sensitivity. Further, through the verification of the present invention, the fusion enzyme obtained by the above design idea can not only achieve good enzyme activity stability, but also significantly improved the detection sensitivity of a paper-based biosensor manufactured from the fusion enzyme, and the detection effect of the above sensor is not affected by common interferents including ascorbic acid, uric acid and urea in a biological sample. Based on the results of this study, by applying the above fusion enzyme to the development of a paper-based biosensor, it is expected to obtain detection products with more excellent detection performance such as blood sugar and urine sugar.

According to a second aspect of the present invention, provided is use of the fusion enzyme according to the first aspect in the manufacture of a glucose detection element.

The glucose detection element is further a paper-based biosensing element.

A paper-based glucose detection device has the advantages of being simple to assemble and portable in use. Due to the influence of oxygen partial pressure, the traditional glucose oxidase detection element has great limitations in detecting samples with obvious differences in oxygen content, and glucose oxidase is often immobilized by physical adsorption or covalent binding. This immobilization mode is unstable, which will affect the active center of the enzyme molecule and reduce the enzyme activity. The fusion enzyme provided by the present invention adopts glucose dehydrogenase (GDH) with flavin adenine dinucleotide (FAD) as a coenzyme as an enzyme element, which can eliminate the influence of oxygen partial pressure on the determination result. In addition, the above glucose dehydrogenase (GDH) which binds to the carbohydrate binding module can obtain a better binding ability to a paper base, effectively reducing the probability that the conventional glucose oxidase is detached from a carrier and the enzyme activity is reduced.

According to a third aspect of the present invention, provided is a paper-based biosensing element, wherein the biosensing element is cellulose paper immobilized with the fusion enzyme according to the first aspect.

According to a fourth aspect of the present invention, provided is a blood glucose test kit, including an immobilized carrier of the fusion enzyme according to the first aspect and/or a paper-based biosensing element according to the third aspect.

The beneficial effects of the above one or more technical solutions are as follows:
currently, a transformation strategy of improving the detection sensitivity of an enzyme electrode sensor by using the specific affinity adsorption characteristics of CBM to cellulose to construct the fusion enzyme, and using the paper base as the immobilized carrier of the fusion enzyme molecule is hardly reported at home and abroad. The present invention provides excellent performance of the fusion enzyme for the first time. Based on the exploration of the cellulose binding specificity of the fusion enzyme, those skilled in the art can easily obtain a variety of glucose level monitoring products with better performance, which is of great significance for glucose level monitoring in a clinical sample and the prevention and treatment of diabetes.

### BRIEF DESCRIPTION OF THE DRAWINGS

The accompanying drawings forming a part of the present invention are intended to provide a further understanding of the present invention, and illustrative examples of the present invention and description thereof are intended to illustrate the present invention and do not constitute an improper limitation of the present invention.

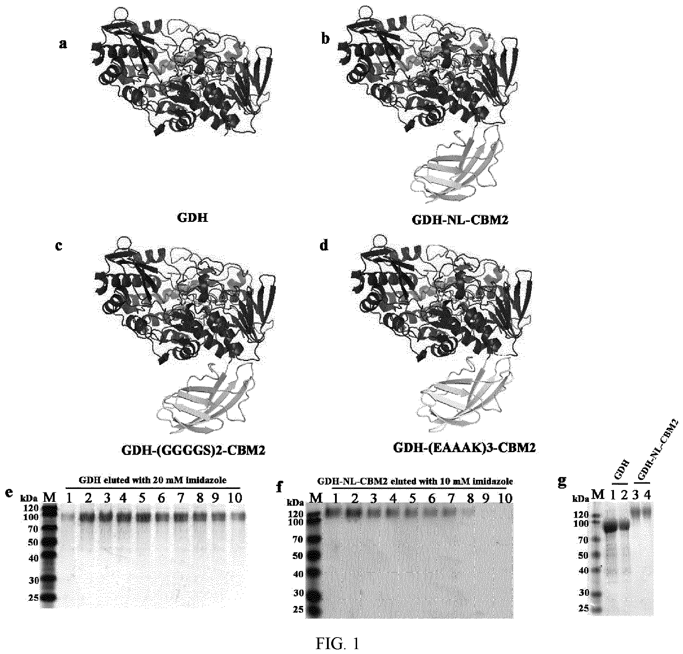

FIG. 1 shows SDS-PAGE detection of heterologously expressed glucose dehydrogenase;
   wherein (a) is a predicted structure of wild-type glucose dehydrogenase (GDH);
   (b), (c), and (d) are predicted structures of fusion glucose dehydrogenase (GDH-NL-CBM2, GDH-(GGGGS)2-CBM2, and GDH-(EAAAK)3-CBM2);
   (e) a band M: a protein standard, and an SDS-PAGE detection pattern of GDH eluted with 20 mM imidazole;
   (f) an SDS-PAGE detection pattern of GDH-NL-CBM2 eluted with 10 mM imidazole; and
   (g) an SDS-PAGE detection pattern of GDH and GDH-NL-CBM2 which are concentrated by ultrafiltration.
FIG. 2 shows the determination of the optimal temperature and pH of GDH and GDH-NL-CBM2;
   (a) is a diagram of the optimum pH determination results; and (b) is a diagram of the optimum temperature determination results.
FIG. 3 is a pattern diagram of a cellulose paper-based biosensor immobilized with GDH-NL-CBM2.
FIG. 4 shows the electrochemical performance test results of GDH and GDH-NL-CBM2/cellulose paper-based biosensors in PBS and different concentrations of glucose solutions;
   wherein (a) and (b) are cyclic voltammetry detection curves of GDH and GDH-NL-CBM2, respectively; (c) and (d) are chronoamperometry detection curves of GDH and GDH-NL-CBM2, respectively; and (e) shows the analysis of linear response of GDH and GDH-NL-CBM2 to glucose.

### DETAILED DESCRIPTION

It should be noted that the following detailed description is illustrative and is intended to provide further explanation of the present invention. Unless otherwise specified, all technical and scientific terms used herein have the same meaning as commonly understood by those of ordinary skill in the art to which the present invention belongs.

It should be noted that the terms used herein are for the purpose of describing specific embodiments only and are not intended to limit exemplary embodiments according to the present invention. As used herein, a singular form is also intended to include a plural form as well unless the context clearly indicates otherwise. Furthermore, it should also be understood that the terms "comprise" and/or "include", when used in this specification, indicate the presence of features, steps, operations, devices, components and/or combinations thereof.

As introduced in the background, the paper-based glucose sensor has the advantages of portability and simple development, in order to provide a paper-based glucose sensor for glucose dehydrogenase with flavin adenine dinucleotide (FAD) as a coenzyme, the present invention proposes a fusion enzyme in which flavin adenine dinucleotide (FAD)-dependent glucose dehydrogenase (GDH) binds to a carbohydrate binding module (CBM).

According to a first aspect of the present invention, provided is a fusion enzyme, including a peptide fragment of glucose dehydrogenase (GDH) and a peptide fragment of a carbohydrate binding module (CBM), the two parts being linked by a flexible peptide chain.

Preferably, the peptide fragment of glucose dehydrogenase is derived from Aspergillus niger An76.

Further, an amino acid sequence of the GDH is as shown in SEQ ID NO: 1, or amino acid sequences having a similarity of 80% or more with an amino acid sequence shown in SEQ ID NO: 1, and having the same functions as the amino acid sequence shown in SEQ ID NO: 1 are also within the protection scope of the present invention.

Still further, the present invention also provides a coding gene of the amino acid sequence shown in SEQ ID NO: 1, having a sequence shown in SEQ ID NO: 2; and in addition to this gene sequence, gene sequences which can also be translated into the amino acid sequence shown in SEQ ID NO: 1 because of codon degeneracy are also within the protection scope of the present invention because the same technical effect can be realized.

Preferably, the peptide fragment of the carbohydrate binding module (CBM) is selected from one of CBM1, CBM2, CBM3, CBM5, and CBM10.

Further, the carbohydrate binding module is a family 2 CBM (CBM2) having a sequence as shown in SEQ ID NO: 3, or amino acid sequences having a similarity of 80% or more with an amino acid sequence shown in SEQ ID NO: 3, and having the same functions as the amino acid sequence shown in SEQ ID NO: 3 are also within the protection scope of the present invention.

Still further, the present invention also provides a coding gene of the amino acid sequence shown in SEQ ID NO: 3, having a sequence shown in SEQ ID NO: 4; and in addition to this gene sequence, gene sequences which can also be translated into the amino acid sequence shown in SEQ ID NO: 3 because of codon degeneracy are also within the protection scope of the present invention because the same technical effect can be realized.

Preferably, the flexible peptide chain is a linker peptide selected from one or more of (EAAAK)3, (GGGGS)2 and a native linker sequence (NL) linking a catalytic domain with a CBM2 domain in endo-β-xylanase (EM_PRO: Z81013.1) in the genome of Thermobifida fusca.

In a solution with better effects validated by the present invention, the linker peptide is selected from the native linker sequence (NL) linking the catalytic domain with the CBM2 domain in endo-β-xylanase (EM_PRO: Z81013.1) in the genome of Thermobifida fusca. Through verification, fusion expression of GDH and carbohydrate-binding modules (CBMs) by addition of the linker peptide may allow direct binding of GDH to cellulose paper materials, and may allow the active center of GDH to be unhidden, maintaining high activity, thereby ensuring the detection sensitivity of the glucose sensor.

In other embodiments, the present invention also provides a linker peptide (EAAAK)3 shown in SEQ ID NO: 7 and its corresponding coding gene (SEQ ID NO: 8), and a linker peptide (GGGGS)2 shown in SEQ ID NO: 9 and its corresponding coding gene (SEQ ID NO: 10).

Further, a native linker peptide sequence of the CBM2 domain is shown in SEQ ID NO: 5, or amino acid sequences having a similarity of 80% or more with an amino acid sequence shown in SEQ ID NO: 5, and having the same functions as the amino acid sequence shown in SEQ ID NO: 5 are also within the protection scope of the present invention.

Still further, the present invention also provides a coding gene of the amino acid sequence shown in SEQ ID NO: 5, having a sequence shown in SEQ ID NO: 6; and in addition to this gene sequence, gene sequences which can also be translated into the amino acid sequence shown in SEQ ID NO: 5 because of codon degeneracy are also within the protection scope of the present invention because the same technical effect can be realized.

In one specific embodiment of the above preferred technical solution, the amino acid sequence of the fusion enzyme from a N-terminus to a C-terminus is shown in SEQ ID NO: 11. Meanwhile, the present invention also provides a coding gene of the above fusion protein, wherein the coding gene of the fusion protein has a sequence shown in SEQ ID NO: 12.

In other embodiments, provided are a fusion enzyme GDH-(EAAAK)3-CBM2 (having an amino acid sequence shown in SEQ ID NO: 13) and its corresponding coding gene (SEQ ID NO: 14), and a fusion enzyme GDH-(GGGGS)2-CBM2 (having an amino acid sequence shown in SEQ ID NO: 15) and its corresponding coding gene (SEQ ID NO: 16).

In addition, an expression method of the fusion enzyme described above can be achieved by linking polynucleotide into a vector or by integrating polynucleotide into the genome of a host cell. As used herein, the term "vector" refers to a DNA construct including a nucleotide sequence encoding a desired protein, which is operably linked to an appropriate expression regulatory sequence to express the desired protein in a suitable host cell. The regulatory sequence includes a promoter that can initiate transcription, an optional operator sequence that regulates transcription, a sequence encoding a suitable mRNA ribosome binding site, and a sequence which regulates the termination of transcription and translation. After a vector is transformed into an appropriate host cell, the vector can replicate or function independently of the host genome, and the vector can be integrated into the genome itself.

Further, the present invention provides a specific preparation method, namely the step of transforming a coding gene into Pichia pastoris, followed by expression and purification.

The vector used in the present invention is not particularly limited as long as it can replicate in a host cell, and any known vector in the art may be used. Examples of conventional vectors may include natural or recombinant plasmids, cosmids, viruses and phages. For example, pWE15, M13, MBL3, MBL4, IXII, ASHII, APII, t10, t11, Charon 4A and Charon 21A may be used as phage vectors or cosmid vectors. As the plasmid vectors, a pBR type, a pUC type, a pBluescriptII type, a pGEM type, a pTZ type, a pCL type and a pET type can be used. The vector that can be used in the present invention is not particularly limited, and any known expression vector can be used. Preferably, a pDZ vector, a pPIC9k vector, a pACYC184 vector, a pCL vector, a pECCG117 vector, a pUC57 vector, a pBR322 vector, a pMW118 vector or pCCIBAC vector may be used.

According to a second aspect of the present invention, provided is use of the fusion enzyme according to the first aspect in the manufacture of a glucose detection element.

Preferably, the glucose detection element is a paper-based biosensing element.

According to a third aspect of the present invention, provided is a paper-based biosensing element, wherein the biosensing element is cellulose paper immobilized with the fusion enzyme according to the first aspect.

Preferably, a preparation method for the paper-based biosensing element includes the following steps of: immobilizing the fusion enzyme on a cellulose paper base, and then printing electrodes on the paper base immobilized with the fusion enzyme.

In one specific embodiment, the preparation method includes the following steps of: uniformly mixing polydimethylsiloxane with lanolin by sonicating, printing the mixture on nanocellulose paper to form a hydrophobic region, adding dropwise the fusion enzyme in the first aspect in a working electrode printing region of a hydrophilic region of the nanocellulose paper, performing drying at room temperature, and separately printing the working electrode, a counter electrode and a reference electrode in the hydrophilic region.

According to a fourth aspect of the present invention, provided is a blood glucose test kit, including an immobilized carrier of the fusion enzyme according to the first aspect and/or a paper-based biosensing element according to the third aspect.

Preferably, the blood glucose test kit further includes a blood sample collection device and the like.

In order to enable those skilled in the art to understand the technical solutions of the present invention more clearly, the technical solutions of the present invention will be described in detail below in conjunction with specific examples.

### Example 1 Fusion expression of CBM and GDH

The sequences of CBMs with binding specificity for cellulose were selected from CBM1, CBM2, CBM3, CBM5, and CBM10 families, and the amino acid frequencies, functional framework sequence profiles and other information of different CBMs were statistically analyzed by using structural bioinformatics analysis tools (SWISS-MODEL, ClustalX, VMD and PyMOl software) to screen CBM2 in Thermobifida fusca for fusion enzyme construction.

Based on the analysis of the structural characteristics of CBM2 and GDH, linker sequences ((GGGGS)2 and (EAAAK)3) for a fusion enzyme molecule were selected from the LinkerDB database, while a native linker sequence linking a catalytic domain and a CBM2 domain in endo-β-xylanase (EM_PRO: Z81013.1) in the genome of Thermobifida fusca was selected; and the linker sequences and the native linker sequence were respectively used as a linker peptide for the construction of a GDH fusion enzyme, the native linker sequence is "LGGDSSGGGPGEPGGPGGPGEPGGPGGPGEPGGPGDGT", and predicted structures of GDH and GDH fusion enzymes are shown in FIGS. 1a-1d.

### Example 2

Primers were designed by using a pPIC9k-GDH plasmid linked to a codon-optimized Aspergillus niger An76 GDH-encoding gene obtained in the previous study as a template to obtain a gene encoding GDH, and primers were designed by using a plasmid pUC57-linker-CBM2 DNA as a template to obtain a gene encoding a linker as well as CBM2. Three GDH fusion enzyme genes with different linker peptides were obtained according to a method of a Homologous Recombination Kit from Nanjing Vazyme, and the three GDH fusion enzyme genes respectively use a pPIC9K plasmid as an expression vector and Pichia pastoris GS115 as an expression host to achieve GDH fusion enzyme expression.

### (1) GDH fusion enzyme gene cloning:

By using the principle of homologous recombination and a sequence near a cloning site of Pichia pastoris GS115, six pairs of primers were designed from a codon-optimized GDH gene sequence and a pUC57-linker-CBM2 gene sequence, respectively:

| | GDH fusion enzyme with a native linker as a linker peptide |
|---|---|
| P1 | 5'-gctgaagcttacgtagaattcggttctccagctcattacgatttt-3' |
| P2 | 5'-cgccgaggtaagattccttaatcaaatcggaagc-3' |
| P3 | 5'-taaggaatcttacctcggcggcgactcctcc-3' |
| P4 | 5'-aaggcgaattaattcgcggccgcttagtggtggtggtggtggtg-3' |

| | GDH fusion enzyme with (GGGGS)2 as a linker peptide |
|---|---|
| P1' | 5'-gctgaagcttacgtagaattcggttctccagctcattacgatttt-3' |
| P2' | 5'-acctccaccgtaagattccttaatcaaatcggaagc-3' |
| P3' | 5'-aggaatcttacggtggaggtggatctggagg-3' |
| P4' | 5'-aaggcgaattaattcgcggccgctcaggagcaggtggctccg-3' |

| | GDH fusion enzyme with (EAAAK)3 as a linker peptide |
|---|---|
| P1" | 5'-gctgaagcttacgtagaattcggttctccagctcattacgatttt-3' |
| P2" | 5'-ggcagcttcgtaagattccttaatcaaatcggaagc-3' |
| P3" | 5'-aggaatcttacgaagctgccgcaaaagaagc-3' |
| P4" | 5'-aaggcgaattaattcgcggccgctcaggagcaggtggctccg-3' |

PCR amplification was performed by using the pPIC9K-GDH plasmid linked to the codon-optimized Aspergillus niger An76 GDH-encoding gene as a template and respectively using P1, P2, P1', P2', P1", and P2" as primers to obtain GDH genes with different homologous recombination arms, and PCR amplification was performed by using a pUC57-linker-CBM2 gene as a template, and using P3, P4, P3', P4', P3", and P4" as primers to obtain genes encoding different linker peptides and CBM2. The PCR reaction was carried out in a 50 µI system (referring to Phanta^{®}Max Super-Fidelity DNA Polymerase) under the conditions of 30 cycles of pre-denaturation at 95°C for 3 min, denaturation at 95°C for 15 s, annealing at 60°C for 15 s, and extension at 72°C for 1 min, followed by extension at 72°C for 5 min. PCR fragments of the GDH gene and the linker-CBM2 gene were amplified separately, and recovered by gel cutting (purification by a product purification kit). The PCR fragments of the GDH gene and the linker-CBM2 gene which are recovered by gel cutting were then ligated onto a pPIC9k plasmid vector by using the ClonExpress^{®}Ultra One Step Cloning Kit product from Nanjing Vazyme to be uniformly mixed by pipetting in a 10 µl reaction system (1 µl of a linearized vector, 2 µl of a PCR fragment of the GDH gene, 1.5 µl of a PCR fragment of a linker-CBM1 gene, 5 µl of 2×ClonExpress Mix, and 0.5 µl of ddH2O), the mixture was reacted at 50°C for 15 min, and the resulting reaction solution was immediately cooled on ice to obtain a pPIC9k-GDH-linker-CBM2 fusion enzyme gene recombination product.

### (3) Plasmid enrichment by transformation of pPIC9k-GDH-linker-CBM2 fusion enzyme gene into Escherichia coli

The pPIC9k-GDH-linker-CBM2 fusion enzyme gene recombination product was mixed with Escherichia coli DH5α, and the mixture was heat shocked for 90 s, plated on a 100 µg/mL ampicillin-resistant LB agar culture plate, and incubated overnight at 37°C. Single colonies were picked and then a plasmid was extracted for electrophoresis detection, and the plasmid was stored at -20°C. A target fragment was then detected by EcoRI and Notl digestion, then a bacterial suspension was sent for sequencing by a company, and the correctly sequenced plasmid was transformed into Escherichia coli in the same way to achieve plasmid enrichment.

### (4) Transformation of pPIC9k-GDH-linker-CBM2 into Pichia pastoris host strain

A single colony of Pichia pastoris GS115 was inoculated into a test tube containing 5 mL of a YPD liquid medium, and incubated overnight at 30°C. The incubated single colony was transferred to a conical flask containing 50 mL of a YPD liquid medium in a 1% inoculation amount, and incubated overnight at 30°C until OD600=1.3-1.5; the culture solution was centrifuged at 1500 g at 4°C for 5 min, the supernatant was discarded, and the cells were resuspended in 50 mL of ice-bathed double distilled water; the culture solution was centrifuged at 1500 g at 4°C for 5 min, the supernatant was discarded, and the cells were resuspended in 25 mL of ice-bathed double distilled water; the culture solution was centrifuged at 1500 g at 4°C for 5 min, the supernatant was discarded, and the cells were resuspended in 2 mL of an ice-bathed 1 M sorbitol solution; the culture solution was centrifuged at 1500 g at 4°C for 5 min, the supernatant was discarded, and the cells were resuspended in 100 µl of an ice-bathed 1 M sorbitol solution to make the volume of the bacterial suspension be in approximately 150 µl; and 80 µl of treated competent cells and 5-20 µg of a pPIC9k-GDH-linker-CBM2 plasmid linearized with bg1I were added to a 1.5 mL precooled centrifuge tube, and uniformly mixed. The mixed solution was then transferred into a pre-ice-bathed transformation cup (a 0.2 cm type); the transformation cup containing the transformation mixed solution was ice-bathed for 5 min; an electroporator was set according to biorad Pichia pastoris electroporation parameters, and an electric pulse was started, after the pulse, 1 mL of an ice-bathed 1 M sorbitol solution was immediately added to the transformation cup, and then the transformation solution was transferred to a new 1.5 mL centrifuge tube; and incubated at 30°C for 2 h. 100 µL of a GS115 transformation solution was pipetted and plated onto an MD plate, and incubated at 30°C until a transformant appeared. Colony PCR verification was performed on single clones transformed on the MD plate, ensuring integration of exogenous genes.

### (5) Induced expression of GDH-linker-CBM2 fusion enzyme gene

The selected Pichia pastoris recombinant was inoculated into 5 mL of a BMGY liquid medium, and incubated with shaking overnight at 30°C at 250 rpm; 500 µL of the overnight culture was transferred to 50 mL of a BMGY liquid medium, and incubated with shaking at 30°C at 250 rpm until OD600=2-6 (a logarithmic growth phase, approximately 16-18 h); centrifugation was performed at 3000 g for 5 min, the supernatant was discarded, the cells were resuspended with a BMMY liquid medium until OD600=1.0, a final concentration of methanol being 1%, the obtained cell suspension was placed in a 500 mL conical flask, and the conical flask was sealed with 8 layers of sterile gauze, and the cells were incubated with shaking at 30°C at 250 rpm for 5 d; a fermentation broth was centrifuged at 8000 rpm to obtain a crude enzyme solution, the expression of a protein of interest in the crude enzyme solution was detected by SDS-PAGE, and an enzyme activity assay was performed. The results showed that fusion enzymes with (GGGGS)2, (EAAAK)3, and the native linker sequence (NL) as linker peptides, respectively were expressed in the crude enzyme solution, but there was a difference in enzyme activity, with the highest enzyme activity (2480 U/L) in the fermentation broth of the GDH fusion enzyme with NL as the linker peptide, and the enzyme activities in the fermentation broths of the GDH fusion enzymes with (GGGGS)2 and (EAAAK)3 as the linker peptides were 1860 U/L and 1520 U/L, respectively.

### (6) Isolation and purification of GDH-linker-CBM2 fusion enzyme

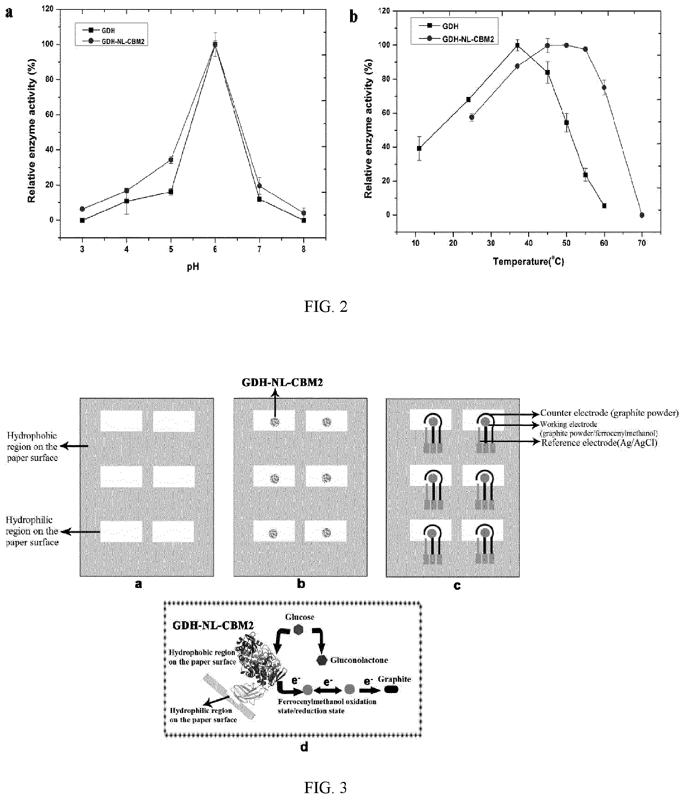

The crude enzyme solutions of the GDH fusion enzymes linked by the three linker peptides were respectively mixed with a nickel-containing filler to be bound in a refrigerator with rotation at 4°C for 6 h. The protein of interest was then eluted with a gradient concentration of imidazole solutions, and the protein solution eluted with 20 mM imidazole is ultrafiltered by using a 3 K ultrafiltration tube using a disodium hydrogen phosphate-citric acid buffer having a pH of 5.0 at 4900 rpm at 4°C until the pH of the buffer flowing down was 5.0, the ultrafiltration was stopped, and the GDH fusion enzyme solution obtained by ultrafiltration was collected, and was subjected to an enzyme activity assay and SDS-PAGE to detect the protein of interest. The results showed that the fusion enzyme with (GGGGS)2 as the linker peptide may not be exposed normally due to a 6×His purification tag, as a result, the purified fusion enzyme could not be obtained, and the enzyme activity (2360 U/L) of the purified fusion enzyme with (EAAAK)3 as the linker peptide was about 1/4 of the activity (92000 U/L) of the fusion enzyme with the native linker sequence as the linker peptide, thus the GDH fusion enzyme constructed with the native linker sequence as the linker peptide had a clear enzyme activity advantage, and its properties were further tested for analysis. The SDS-PAGE detection pattern showed that the molecular weight of GDH-NL-CBM2 was significantly greater than that of wild-type GDH (FIGS. 1e-1g), indicating that CBM2 was successfully fused to the GDH enzyme molecule and that the molecular weight of the actually expressed enzyme molecules was higher than the predicted molecular weight due to glycosylation of the enzyme molecule. The optimum pH of the fusion enzyme was determined to be 6.0, and the optimum reaction temperature of the fusion enzyme was determined to be 50°C. Compared with wild-type GDH (pH 6.0, 37°C), there was no significant change in the optimum pH, while the optimum reaction temperature was significantly increased. This may be due to the fact that CBM2 is derived from Thermobifida fusca, has higher temperature tolerance, and fusion of CBM2 into the enzyme molecule increases the temperature adaptability of the entire enzyme molecule (FIG. 2).

### Example 3 Comparison of the detection performance of paper-based sensing elements manufactured from GDH and GDH-NL-CBM2 fusion enzyme

Polydimethylsiloxane and lanolin were uniformly mixed in a ratio of 10: 1 by sonicating at 60 Hz for 30 min, and the mixture was printed on the prepared nanocellulose paper having a pore size of 0.1 µm to form a hydrophobic region. A GDH wild-type enzyme molecule (10 U, 30 µL) isolated and purified in the previous laboratory study, and a mixed solution of 25% glutaraldehyde (2 µL) and 0.5% chitosan (8 µL), and 40 µL of the GDH-NL-CBM2 fusion enzyme (10 U) were added dropwise in a working electrode printing region of a hydrophilic region of the nanocellulose paper, and drying was performed at room temperature for 4 h. A working electrode, a counter electrode and a reference electrode were then printed separately in the hydrophilic region (FIG. 3). The performance of the sensing elements manufactured from the GDH wild-type enzyme and the GDH-NL-CBM2 fusion enzyme was tested separately by using a three-electrode system of an electrochemical workstation.

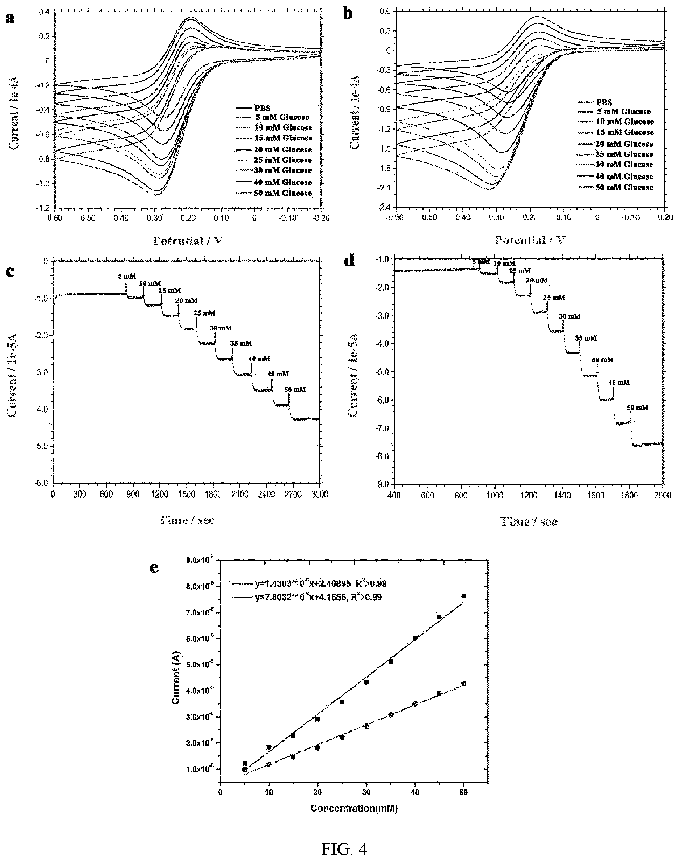

The paper-based enzyme sensing elements were sequentially scanned and detected by cyclic voltammetry in a PBS buffer having a pH of 7.0 and glucose solutions of different concentrations (5 mM, 10 mM, 15 mM, 20 mM, 25 mM, 30 mM, 35 mM, 40 mM, and 50 mM) at a potential ranging from -0.2 V to 0.6 V The results showed that for both paper-based enzyme sensing elements, a pair of distinct redox peaks of ferrocene can be detected in the PBS buffer, and a current value corresponding to an oxidation peak potential (+0.26 V) gradually increased with the increase of the glucose concentration, while a current value corresponding to a reduction peak potential (+0.17 V) gradually decreased (FIGS. 4a and 4b), indicating that the paper-based enzyme sensing elements in both immobilization modes have glucose electrocatalytic properties. The glucose linear detection range was further explored by using chronoamperometry, and the results showed current changes in both paper-based enzyme sensing elements were linearly correlated (R2≥0.99) within the range of glucose concentrations from 5 mM to 50 mM (FIGS. 4c and 4d), but the sensing element manufactured from the GDH-NL-CBM2 fusion enzyme had a higher sensitivity (20.23 µA.mM-1.cm-2) and a lower detection limit of 2.53 mM (S/N=3) compared with the detection performance of the sensing element manufactured from the GDH enzyme (a sensitivity of 10.75 µA.mM-1.cm-2, and a detection limit of 2.62 mM) (4e). Meanwhile, a sensor with GDH as a sensing element greatly improves the interference resistance against substances such as ascorbic acid, uric acid, urea, etc., compared with a sensor with glucose oxidase as a sensing element.

The paper-based sensing element manufactured from the GDH-NL-CBM2 fusion enzyme has the characteristics of simple manufacturing and better detection performance compared with the sensing element manufactured from the GDH, and therefore, the paper-based sensing element manufactured from the GDH-NL-CBM2 fusion enzyme has broad application prospects in the field of biosensors.

The above are only the preferred examples of the present invention, and are not intended to limit the present invention, and for those skilled in the art, various modifications and variations of the present invention may be made. Any modification, equivalent replacement, improvement and the like made within the spirit and principle of the present invention should be included within the protection scope of the present invention.

## Claims

1. A fusion enzyme, **characterized by** comprising a peptide fragment of glucose dehydrogenase and a peptide fragment of a carbohydrate binding module, the two parts being linked by a flexible peptide chain.

2. The fusion enzyme according to claim 1, **characterized in that** the peptide fragment of glucose dehydrogenase is derived from *Aspergillus niger* An76;
preferably, an amino acid sequence of the GDH is as shown in SEQ ID NO: 1 or amino acid sequences having a similarity of 80% or more with an amino acid sequence shown in SEQ ID NO: 1, and having the same functions as the amino acid sequence shown in SEQ ID NO: 1 are also within the protection scope of the present invention; and
further, a coding gene of the amino acid sequence shown in SEQ ID NO: 1 has a sequence shown in SEQ ID NO: 2; and in addition to this gene sequence, gene sequences which can also be translated into the amino acid sequence shown in SEQ ID NO: 1 because of codon degeneracy are further comprised.

3. The fusion enzyme according to claim 1, **characterized in that** the peptide fragment of the carbohydrate binding module is selected from one of CBM1, CBM2, CBM3, CBM5, and CBM10.

4. The fusion enzyme according to claim 3, **characterized in that** the carbohydrate binding module is a family 2 CBM, wherein a sequence of the CBM2 is as shown in SEQ ID NO: 3, or is an amino acid sequence having a similarity of 80% or more with an amino acid sequence shown in SEQ ID NO: 3, and having the same functions as the amino acid sequence shown in SEQ ID NO: 3; and
preferably, further provided is a coding gene of the amino acid sequence shown in SEQ ID NO: 3, having a sequence as shown in SEQ ID NO: 4; and in addition to this gene sequence, gene sequences which can also be translated into the amino acid sequence shown in SEQ ID NO: 3 because of codon degeneracy are further comprised.

5. The fusion enzyme according to claim 1, **characterized in that** the flexible peptide chain is a linker peptide selected from one or more of (GGGGS)2, (EAAAK)3, and a native linker sequence linking a catalytic domain with a CBM2 domain in endo-β-xylanase in the genome of *Thermobifida fusca.*

6. The fusion enzyme according to claim 5, **characterized in that** the linker peptide is selected from the native linker sequence linking the catalytic domain and the CBM2 domain in endo-β-xylanase in the genome of *Thermobifida fusca*;
preferably, a sequence of the linker peptide is as shown in SEQ ID NO: 5, or is an amino acid sequence having a similarity of 80% or more with an amino acid sequence shown in SEQ ID NO: 5, and having the same functions as the amino acid sequence shown in SEQ ID NO: 5; and
further, further provided is a coding gene of the amino acid sequence shown in SEQ ID NO: 5, having a sequence shown in SEQ ID NO: 6; and in addition to this gene sequence, gene sequences which can also be translated into the amino acid sequence shown in SEQ ID NO: 5 because of codon degeneracy are further comprised.

7. The fusion enzyme according to claim 1, **characterized in that** an amino acid sequence of the fusion enzyme from a N-terminus to a C-terminus is shown in SEQ ID NO: 11; and a coding gene of the fusion enzyme is shown in SEQ ID NO: 12;
or, an amino acid sequence of the fusion enzyme is as shown in SEQ ID NO: 13 or SEQ ID NO: 15;
preferably, the fusion enzyme is prepared by transforming a coding gene shown in SEQ ID NO: 12 into *Pichia pastoris,* followed by expression and purification.

8. Use of the fusion enzyme according to any one of claims 1-7 in the manufacture of a glucose detection element, wherein the glucose detection element is a paper-based biosensing element.

9. A paper-based biosensing element, **characterized in that** the biosensing element is cellulose paper immobilized with the fusion enzyme according to any one of claims 1-7;
preferably, a preparation method for the paper-based biosensing element comprises the following steps of: immobilizing the fusion enzyme on a cellulose paper base, and then printing electrodes on the paper base immobilized with the fusion enzyme; and
further, the preparation method comprises the following steps of: uniformly mixing polydimethylsiloxane with lanolin by sonicating, printing the mixture on nanocellulose paper to form a hydrophobic region, adding dropwise the fusion enzyme in the first aspect in a working electrode printing region of a hydrophilic region of the nanocellulose paper, performing drying at room temperature, and separately printing the working electrode, a counter electrode and a reference electrode in the hydrophilic region.

10. A blood glucose test kit, **characterized by** comprising an immobilized carrier of the fusion enzyme according to any one of claims 1-7 and/or the paper-based biosensing element according to claim 9; wherein
preferably, the blood glucose test kit further comprises a blood sample collection device.
